# EUROPEAN PATENT SPECIFICATION

(11) **EP 1 318 821 B1**
(45) Date of publication and mention of the grant of the patent: **04.06.2008**
(21) Application number: 01979074.0
(22) Date of filing: 21.09.2001
(51) Int. Cl.: A61K 33/14, A61P 13/12

(54) **FLUID FOR HAEMOFILTRATION**
FLÜSSIGKEIT FÜR DIE HÄMOFILTRATION
LIQUIDE POUR HEMOFILTRATION

(30) Priority: 21.09.2000 NL 1016235
(43) Date of publication of application: 18.06.2003
(73) Proprietor: Hollander,, Adrianus Antonius Marcus Johannes, 5263 EX Vught (NL)
(72) Inventor: Hollander,, Adrianus Antonius Marcus Johannes, 5263 EX Vught (NL)
(74) Representative: Jorritsma, Ruurd
(86) International application number: PCT/NL2001/000698
(87) International publication number: WO 2002/024209

(56) References cited:
- DE-A- 4 122 754
- C.J.OLBRIGHT: "Substitutionslösungen zur kontinuierlichen Hämofiltration" INTENSIVMEDIZIN UND NOTFALLMEDIZIN, vol. 32, no. 3, 1995, pages 194-194-198, XP001002227

## Description

The present invention relates to a fluid suitable for use in haemofiltration, in particular for use in continuous veno-venous haemofiltration (CVVH). The fluid comprises an aqueous solution of physiologically acceptable salts containing at least the Na⁺, Cl⁻, Mg²⁺, K⁺ and Ca²⁺ ions and optionally glucose, all in a physiologically acceptable concentration.

Dialysis is the indicated treatment for patients whose kidney function is failing. The removal of waste substances from the blood is effected by transfer to an external fluid or replacement of plasma liquid by an external fluid. Various dialysis techniques, with associated dialysis fluids, can be differentiated, which are used depending on the type of patient. In the case of patients suffering from long-term renal insufficiency, the dialysis technique used is usually an intermittent treatment a few times (2 to 3 times) per week for a few hours (3 to 5 hours). With this technique, known as haemodialysis, waste substances, in particular urea, salts and other small molecules, are removed from the blood by means of diffusion through a semi-permeable membrane. Another form of dialysis is peritoneal dialysis. In contrast to haemodialysis, where the blood is passed over a dialysis fluid through a dialysis unit (artificial kidney) outside the body, in the case of peritoneal dialysis a dialysis fluid is introduced into a patient's abdominal cavity (peritoneum), the peritoneum acting as semi-permeable membrane.

In the case of patients suffering from acute renal insufficiency, continuous treatment, throughout the entire day for several days to several weeks, is the indicated treatment. A technique other than haemodialysis, specifically haemofiltration, is used for this. In the case of haemofiltration, waste substances are removed from the blood by means of convective flow through a highly permeable membrane. In this way the abovementioned waste substances are removed in larger amounts and large(r) molecules are also removed. In addition, in the case of haemofiltration an appreciable quantity of liquid, which can vary from 1 to 5 litres per hour, is removed from the bloodstream. In contrast to haemodialysis, this demands that in the case of haemofiltration a replacement fluid must be returned to the patient in large quantities. Optionally a combination of dialysis and filtration can be used. This is termed haemodiafiltration. A specific type of haemofiltration is continuous veno-venous haemofiltration, abbreviated as CVVH. This specific type is discussed further below in the description.

As a consequence of the nature and the importance of the field of application, many dialysis fluids are known, based on decades of research.

In a review article, Forni and Hilton, N. Eng. J. Med. 1997, vol. 236, 1003-1309, describe the basic principles of haemofiltration and compare this with haemodialysis. It can clearly be seen from this article that in the case of acute renal failure haemofiltration is to be preferred to haemodialysis. The authors also describe a characterising composition of a replacement fluid for haemofiltration. This fluid contains the usual components Na⁺, Cl⁻, Mg²⁺ and glucose and, as buffer, lactate. The fluid also contains Ca²⁺ in a concentration of 1.6 mmol/l. There is no K⁺ in the fluid beforehand. Immediately before use, K⁺ in the form of potassium chloride must be added in a concentration of up to 4 mmol/l.

A few examples of commercially available haemofiltration fluids are Sifra BH 504 (Direnco), Schiwa SH21 (Direnco) and Monosol-K B9101 (Baxter). In addition to Na⁺, Cl⁻, Mg²⁺ and, as buffer, lactate, these haemofiltration fluids contain 1.5 mmol/l K⁺, 1.5 mmol/l Ca²⁺ and 11.1 mmol/l glucose (Sifra BH 504), 4 mmol/l K⁺, 2 mmol/l Ca²⁺ and no glucose (Schiwa SH21) and 4 mmol/l K⁺, 1.75 mmol/l Ca²⁺ and approximately 5 mmol/l glucose (Monosol-K B9101).

DE 3 917 251 discloses a haemodialysis or peritoneal dialysis fluid buffered by bicarbonate which in addition to the usual components Na⁺, Cl⁻, Mg²⁺ and glucose contains the components K⁺ in a concentration of 0-4 mmol/l and Ca²⁺ in a concentration of 0.5-5 mmol/l. A specific example for peritoneal dialysis is described in which no K⁺ is present and Ca²⁺ is present in a concentration of 1.5 mmol/l.

Dialysis fluids for peritoneal dialysis and replacement fluids for haemofiltration and haemodialysis which contain sodium pyruvate as buffering compound are described in EP 0 658 353. The composition that is described for replacement fluid contains, in addition to the components Na⁺, Cl⁻, Mg²⁺ and pyruvate, K⁺ in a concentration of 0-4 mmol/l and Ca²⁺ in a concentration of 1.0-2 mmol/l. A composition containing 0 mmol/l K⁺ and 1.5 mmol/l Ca²⁺ is given as an example.

EP 0 347 274 describes an infusion and dialysis fluid containing bicarbonate and calcium ions. In addition to bicarbonate, an aqueous acid or a mixture of aqueous acids is present, so that a certain CO₂ pressure is maintained, as a result of which the precipitation of CaCO₃ is prevented. Possible dialysis fluids that are described are compositions which contain, in addition to Na⁺, Cl⁻, Mg²⁺, HCO₃⁻ and acids, the components K⁺ in a concentration of 0-5 mmol/l and Ca²⁺ in a concentration of 0.5-2.5 mmol/l. A specific example in which no K⁺ is present and the Ca²⁺ concentration is 1.75 mmol/l is described for peritoneal dialysis only. In this example the fluid also contains 15 mmol/l glucose.

In EP 0 613 688 dialysis fluids for haemodialysis are described which are obtained from a method in which a first concentrated solution which contains mainly Na⁺, Cl⁻ and HCO₃⁻ and optionally acetate and a second concentrated solution which contains mainly Mg²⁺, K⁺ and Ca²⁺ and optionally glucose are mixed. A concentration of 0-5 mmol/l is cited as the maximum range for K⁺ and a concentration of 0.3-2.5 mmol/l as the maximum range for Ca²⁺ in the dialysis fluids obtainable in accordance with the method. 1-4 mmol/l, with an average of 2 mmol/l, is described as the preferred range for K⁺. For Ca²⁺ the preferred range is 0.75-2 mmol/l, with an average of 1.6 mmol/l. A few examples in EP 0 613 688 describe dialysis fluids having a K⁺ concentration of 3, 4 and 5 mmol/l. In these examples the Ca²⁺ concentration is, respectively, 1.75, 2 and 2 mmol/l. An example is given in which a K⁺ gradient is used, specifically from 4 mmol/l decreasing to 1 mmol/l. In this example the Ca²⁺ concentration is 1.65 mmol/1. With one exception, all dialysis fluids in the examples in EP 0 613 688 contain a Ca²⁺ concentration that is equal to or greater than 1.65 mmol/l. In the single exception the Ca²⁺ concentration is 1.25 mmol/l, but in this case the K⁺ concentration is 1 mmol/l.

As can be seen from all examples in EP 0 613 688, a higher K⁺ concentration is associated with a higher Ca²⁺ concentration. For a person skilled in the art it can be deduced from this that relatively low K⁺ and Ca²⁺ concentrations go together and likewise that relatively high K⁺ and Ca²⁺ concentrations go together. There is not one specific example in the prior art that teaches the simultaneous presence of a relatively high K⁺ and a relatively low Ca²⁺ concentration. DE 4122754 A discloses a solution for haemofiltration comprising Na⁺, Cl⁻, Mg²⁺, K⁺ (1-4 mmol/l) and Ca²⁺ (1.5 mmol/l).

A disadvantage of the dialysis fluids of the prior art is that they are not optimally suited as such for use in haemofiltration. In view of the quantity of fluid that has to be returned in haemofiltration, it is important that this fluid contains physiologically acceptable concentrations of components usually present in the blood, or at least that physiologically acceptable concentrations of components usually present in the blood are obtained when the fluid is used for haemofiltration. This is not the case with the known dialysis fluids, with the consequence that the known dialysis fluids give rise to metabolic disturbances which, in turn, have to be corrected in the patients, who are already under very severe stress.

For instance it has been found that a shortage of potassium, with all the adverse consequences of this, such as loss of strength and disturbances in the heart rhythm, with the risk of a cardiac arrest, occurs when the known dialysis fluids are used in patients. In order to eliminate this shortage and to counteract the adverse consequences, additional potassium is administered to the patient. The additional quantity that is administered is many grams per day. Usually administration is carried out intravenously, which means an additional risk for and stress on the patient. Moreover, risks are associated with the additional administration of such quantities of potassium, such as the occurrence of thrombophlebitis and hyperkalaemia, as a result of which disturbances in the heart rhythm can also occur.

In addition, it has been found that in the case of haemofiltration the use of the known dialysis fluids in patients leads to hypercalcaemia. Too high a concentration of calcium in the blood can lead to impaired conduction and impaired heart rhythm. A situation that is highly disadvantageous for the patient results if it is necessary to stop the haemofiltration treatment prematurely as a consequence of hypercalcaemia.

Hyperglycaemia usually occurs when the known dialysis fluids are used for haemofiltration, which leads to insulin having to be given to these patients. The risk of insulin treatment is that it can result in hypoglycaemia, with the risk of cerebral damage. The hyperglycaemia is partially a consequence of the underlying serious disease, but the too high sugar concentration in the blood is mainly caused by too high a concentration of glucose in the replacement fluids.

The aim of the present invention is to provide a more physiological haemofiltration fluid. The fluid should comprise the components usually present in the blood, including Na⁺, Cl⁻, Mg²⁺, in the form of physiologically acceptable salts, and glucose in such concentrations as are physiologically acceptable, resulting in an optimum fluid for haemofiltration.

The present invention provides this by means of a haemofiltration fluid which is characterised in that the haemofiltration fluid comprises the ions K⁺ in a concentration higher than 3 mmol/l and lower than 5.5 mmol/l and Ca²⁺ in a concentration of 0.8 to 1.3 mmol/l.

With the dialysis fluid according to the present invention it is possible to prevent the occurrence of the adverse consequences of potassium depletion. Moreover, according to the invention the administration of potassium can be dispensed with, as a result of which the additional stress associated with this is avoided, as is the occurrence of the adverse consequences of the administration of additional potassium.

With the dialysis fluids according to the present invention it is also possible to prevent the occurrence of the adverse phenomena of hypercalcaemia and it is also possible to prevent the premature cessation of the treatment as a consequence of hypercalcaemia.

The haemofiltration fluid according to the invention contains components customary for dialysis, including Na⁺, Cl⁻ and Mg²⁺. The components customary for haemofiltration are known to those skilled in the art. For example, in addition to the cited components, glucose is important. The haemofiltration fluid can optionally be supplemented with this. Further components that could be incorporated in the fluid are compounds which are able to influence the pH of the fluid and compounds which are able to influence the clotting of blood. Compounds related to glucose or compounds which are able to replace the function of glucose in the blood can also be incorporated in the haemofiltration fluid. The person skilled in the art will be able to establish whether, and to what extent, such components have to be incorporated in the haemofiltration fluid. According to the invention K⁺ and Ca²⁺ are also present as customary components in haemofiltration fluids.

The components Na⁺, Cl⁻, Mg²⁺, K⁺ and Ca²⁺ and optionally yet further components determined by the person skilled in the art are present in the haemofiltration fluid in the form of a solution, in water, of physiologically acceptable salts. It is known to those skilled in the art which salts are physiologically acceptable. In order to arrive in a practical manner at the composition according to the invention it is preferable to use NaCl, MgCl₂, KCl and CaCl₂, optionally in the form of the hydrate, as physiologically acceptable salts. It is, however, possible to use other physiologically acceptable salts, in particular if it is desired to have components other than those mentioned present in the haemofiltration fluid.

With regard to the concentrations of the components present in the fluid it is clear to those skilled in the art what concentrations are physiologically acceptable. The concentrations of the components are as close as possible to the concentrations that occur in the blood. In this context some spread is, of course, possible, such as occurs from individual to individual. The persons skilled in the art will be able to derive from the concentrations of components occurring in the blood a concentration range within which it is desirable that the concentrations of the customary components will lie in the haemofiltration fluid. For example, the concentrations of the abovementioned customary components Na⁺, Cl⁻, Mg²⁺ will be in the following physiologically acceptable range:

| | | |
|---|---|---|
| Na⁺ | 135-145 | mmol/l |
| Mg²⁺ | 0.6-1.0 | mmol/l |
| Cl⁻ | 95-120 | mmol/l |

The use of known dialysis fluids for haemofiltration appears not to result in physiologically acceptable concentrations of the K⁺ and Ca²⁺ ions present in the blood.

In order to achieve physiologically acceptable concentrations, in the dialysis fluid according to the invention the K⁺ concentration is between 3 and 5.5 mmol/l, preferably between 3.5 and 5 mmol/l. According to the invention the Ca²⁺ concentration in the dialysis fluid is between 0.5 and 1.5 mmol/l, preferably between 0.8 and 1.3 mmol/l.

As has already been mentioned above, in connection with the frequent occurrence of hyperglycaemia in patients it is important to adjust the glucose concentration in the haemofiltration fluid to a suitable value. In one embodiment of the present invention, which is preferred, the glucose concentration is between 0 and 15 mmol/l, preferably between 2 and 10 mmol/l and most preferentially between 3.5 and 8 mmol/l.

In combination with other constituents usually present in the blood, in a preferred embodiment the haemofiltration fluid according to the invention comprises the following components in a physiologically acceptable range, for example:

| | | |
|---|---|---|
| Na⁺ | 135-145 | mmol/l |
| Mg²⁺ | 0.6-1.0 | mmol/l |
| Cl⁻ | 95-120 | mmol/l |
| Glucose | 3.5-8 | mmol/l |
| K⁺ | 3.5-5 | mmol/l |
| Ca²⁺ | 0.8-1.3 | mmol/l |

The above fluid is, for example, suitable for use if haemofiltration is carried out using citrate as anti-clotting agent. In this embodiment blood is treated with citrate upstream of the filter and the replacement fluid can be administered downstream of the filter. Not only does citrate prevent the occurrence of clotting of the blood, as a result of which the filter can become clogged, but citrate also has a buffering action. When citrate is used in haemofiltration it is therefore preferable that the haemofiltration fluid according to the invention does not contain any buffer or contains a low concentration of buffer. Of course, it is advisable to check the pH of the fluid that is returned to the patient, in which context it will be clear to a person skilled in the art whether and how the pH of the fluid has to be corrected, for example by administering bicarbonate solution or, for example, a dilute hydrochloric acid solution.

Depending on the form in which citrate is administered, it is necessary to take into account that the concentrations of the above components remain within the physiological range. For example, it is certainly possible to use an aqueous solution of trisodium citrate for anti-clotting. In this case the replacement fluid should be adjusted and contain fewer sodium ions, so that the Na⁺ concentration that is finally returned to the patient is within the above range. A suitable concentration of trisodium citrate to be used is, for example, between 10 and 15 mmol/l. The haemofiltration fluid according to the invention then should contain 30-45 mmol/l less Na⁺. A person skilled in the art will be able to establish how the haemofiltration fluid has to be adjusted, depending on the form and concentration of citrate used.

In another embodiment it is possible for the citrate for anti-clotting already to be incorporated in the haemofiltration fluid. This replacement fluid is administered upstream of the filter. As has already been stated above, a suitable citrate concentration is between 10 and 15 mmol/l. With this method also the citrate has a buffering action. Since citrate has a calcium-binding action it is desirable to check the quantity of dissolved calcium in the form of Ca²⁺ ions. On the basis of the values found for pH and Ca²⁺ concentration downstream of the filter, a person skilled in the art will be able to correct these by administering pH-correcting solutions mentioned above and/or a further fluid correcting the calcium concentration, such as, for example, haemofiltration fluid according to the invention, so that the calcium concentration that is returned to the patient is within the abovementioned physiologically acceptable range.

For a large proportion of patients it will be possible to use heparin as anti-clotting agent. In this case it is desirable that the haemofiltration fluid contains a buffering compound. Therefore, in addition to the components Na⁺, Cl⁻, Mg²⁺, K⁺, Ca²⁺ and optionally glucose, the fluid according to the invention can also contain one or more buffering compounds. These buffering compounds preferably keep the fluid at physiological pH between 7.2 and 7.6. A weak acid is usually used as buffering compound. Examples of suitable weak acids are lactate, acetate, pyruvate, succinate, fumarate, malate, oxalacetate, oxalsuccinate, α-ketoglutarate and cis-aconitase. Bicarbonate (HCO₃⁻) is also frequently used as buffering compound. It is also possible to use a combination of a weak acid and bicarbonate. In one embodiment of the haemofiltration fluid according to the invention the at least one buffering compound is a weak acid or bicarbonate or a mixture thereof. Lactate or bicarbonate is preferably used as buffering compound according to the invention. In the case of patients suffering from serious liver failure, a lactate-containing replacement fluid can produce lactate acidosis. In this case bicarbonate is preferred as buffering compound. Pyruvate or acetate can also be used as a replacement for lactate.

Depending on the buffer used, according to the invention haemofiltration fluids in the preferred embodiments described below have, for example, the following composition:

| | | |
|---|---|---|
| Na⁺ | 140 | mmol/l |
| Mg²⁺ | 0.8 | mmol/l |
| Cl⁻ | 108 | mmol/l |
| K⁺ | 4 | mmol/l |
| Ca²⁺ | 1.2 | mmol/l |
| Glucose | 6 | mmol/l |
| Lactate | 40 | mmol/l |

and:

| | | |
|---|---|---|
| Na⁺ | 140 | mmol/l |
| Mg²⁺ | 0.8 | mmol/l |
| Cl⁻ | 118 | mmol/l |
| K⁺ | 4 | mmol/l |
| Ca²⁺ | 1.2 | mmol/l |
| Glucose | 6 | mmol/l |
| Bicarbonate | 30 | mmol/l |

The lactate and bicarbonate contents in the above examples ensure that the pH of the fluids is within the physiological range between 7.2 and 7.6. Of course, it is also possible to achieve a pH within the physiological range using concentrations that differ somewhat from the given values. It is clear to those skilled in the art which concentrations of buffering compounds will be suitable in order to obtain the desired pH.

When bicarbonate is used as buffer there is a risk of CaCO₃ precipitating in the fluid before administration to the patient. It is possible to prevent the formation of a precipitate of CaCO₃, for example by mixing bicarbonate with the other components just before use. It is also possible to ensure that bringing Ca²⁺ and HCO₃⁻ ions into contact with one another takes place the shortest possible time before administration to the patient by making use of specific containers for haemofiltration fluids (infusion bags) that consist of several compartments. Since, when mixing the abovementioned components, aqueous solutions, which contain the individual components, are mixed, it will be necessary to adjust the concentrations of the components in the solutions to be combined. Those skilled in the art will be able to determine what the suitable concentrations are in order to fall within the range of the dialysis fluid according to the invention at the point in time when the dialysis fluid is administered to the patient.

The present invention therefore also comprises concentrated haemofiltration fluids with which, after dilution, at the time of administration to a patient, the concentrations of components usually occurring in the blood are within the range of the concentrations of the components according to the invention. It is also possible to prepare a composition of the desired components in dry form in a specific amount, which has to be added to a specific volume of water so that a dialysis fluid is obtained in which the concentrations of the components are within the range of the concentrations of the components according to the invention.

The dialysis fluids according to the invention are suitable for use in a kidney function replacement treatment. In particular they are suitable for use in a continuous kidney function replacement treatment, such as, for example, haemofiltration. Continuous techniques are subdivided into slow continuous ultrafiltration (SCUF), continuous arteriovenous haemofiltration (CAVH) and continuous veno-venous haemofiltration (CVVH). If use is also made of dialysis in addition to filtration, this is termed continuous arteriovenous haemodiafiltration (CAVHD) and continuous veno-venous haemodiafiltration (CVVHD). In the case of CAVH an artery or a vein is punctured and filtration takes place under the influence of the patient's arterial blood pressure. Currently CWH, in which a vein is punctured and the extracorporal circulation with filtration takes place under the influence of a pump, is preferably used. Continuous techniques are indicated in particular in the case of patients suffering acute renal insufficiency or multi-organ failure and in the case of haemodynamic instability, such as, for example, in the case of cardiac patients.

The dialysis fluids according to the invention are used in a manner customary for kidney function replacement treatment. In particular the fluids are used in a manner that is customary for haemofiltration.

The invention is further explained on the basis of the following examples.

### Example 1: Preparation of a composition in non-dissolved form

The following are combined under sterile conditions:

| | | |
|---|---|---|
| NaCl | 5844 g | (100 mol) |
| KCl | 298 g | (4 mol) |
| CaCl₂ dihydrate | 176 g | (1.2 mol) |
| MgCl₂ hexahydrate | 163 g | (0.8 mol) |
| glucose monohydrate | 1189 g | (6 mol) |
| Na L-lactate | 4482 g | (40 mol) |

### Example 2: Preparation of a composition in non-dissolved form

The following are combined under sterile conditions:

| | | |
|---|---|---|
| NaCl | 6248 g | (110 mol) |
| KCl | 298 g | (4 mol) |
| CaCl₂ dihydrate | 176 g | (1.2 mol) |
| MgCl₂ hexahydrate | 163 g | (0.8 mol) |
| glucose monohydrate | 1189 g | (6 mol) |
| NaHCO₃ | 2520 g | (30 mol) |

### Example 3: Preparation of haemofiltration fluid

The composition from Example 1 or 2 is dissolved under sterile conditions in 10001 sterile water, free from electrolytes, and divided between infusion bags.

### Example 4: Preparation of a concentrate

The composition from Example. 1 or 2 is dissolved under sterile conditions in 1001 sterile water, free from electrolytes, and divided between suitable containers. The solution is diluted 10-fold before use.

### Example 5: Preparation of a concentrate

The composition from Example 1 or 2 is dissolved under sterile conditions in 101 sterile water, free from electrolytes, and divided between suitable containers. The solution is diluted 100-fold before use.

### Example 6: Preparation of haemofiltration fluid from composition in non-dissolved form

The well mixed composition from Example 1 is divided into proportionate portions. One hundredth part of the composition, 121.52 g, is dissolved in 101 sterile water, free from electrolytes, before use.

### Example 7: Preparation of haemofiltration fluid from composition in non-dissolved form

The well mixed composition from Example 2 is divided into proportionate portions. One hundredth part of the composition, 107.74 g, is dissolved in 101 sterile water, free from electrolytes, before use.

### Example 8: Preparation of compositions for bicarbonate-buffered haemofiltration fluid and use thereof

For composition I the following are combined under sterile conditions:

| | | |
|---|---|---|
| KCl | 596 g | (8 mol) |
| CaCl₂ dihydrate | 352 g | (2.4 mol) |
| MgCl₂ hexahydrate | 326 g | (1.6 mol) |
| glucose monohydrate | 2378 g | (12 mol) |

For composition II the following are combined under sterile conditions:

| | | |
|---|---|---|
| NaCl | 12856 g | (220 mol) |
| NaHCO₃ | 5040 g | (60 mol) |

a) Compositions I and II are each individually dissolved in 10001 sterile water, free from electrolytes. Proportionate quantities of the solutions of compositions I and II are mixed with one another just before use.
b) Compositions I and II are each individually dissolved in 10001 sterile water, free from electrolyses. The solutions of compositions I and II are distributed in proportionate quantities, separate from one another, between infusion bags having two compartments. The solutions of compositions I and II are mixed with one another at the time of administration.

## Claims

1. Haemofiltration fluid suitable for use in continuous veno-venous haemofiltration (CVVH), that comprises an aqueous solution of physiologically acceptable salts containing at least the Na⁺, Cl⁻, Mg²⁺, K⁺ and Ca²⁺ ions and optionally glucose, all in a physiologically acceptable concentration, wherein the haemofiltration fluid contains the ions K⁺ in a concentration higher than 3 mmol/l and lower than 5.5 mmol/l and Ca²⁺ in a concentration of 0.8 to 1.3 mmol/l.

2. Haemofiltration fluid according to claim 1, wherein the K⁺ concentration is between 3.5 and 5.5 mmol/l, preferably between 3.5 and 5 mmol/l.

3. Haemofiltration fluid according to one of the preceding claims, wherein the glucose concentration is between 0 and 15 mmol/l, preferably between 2 and 10 mmol/l and most preferentially between 3.5 and 8 mmol/l.

4. Haemofiltration fluid according to one of the preceding claims, wherein the fluid comprises the following components in the indicated physiologically acceptable range:
| | | |
|---|---|---|
| Na⁺ | 135 - 145 | mmol/l |
| Mg²⁺ | 0.6 - 1.0 | mmol/l |
| Cl⁻ | 95 - 120 | mmol/l |
| Glucose | 3.5 - 8 | mmol/l |
| K⁺ | 3.5 - 5 | mmol/l |
| Ca²⁺ | 0.8 - 1.3 | mmol/l |

5. Haemofiltration fluid according to one of the preceding claims, wherein the haemofiltration fluid further comprises citrate.

6. Haemofiltration fluid according to claim 5, wherein the citrate concentration is between 10 and 15 mmol/l.

7. Haemofiltration fluid according to one of claims 1 - 4, wherein the fluid further comprises at least one buffering compound.

8. Haemofiltration fluid according to claim 7, wherein the at least one buffering compound is a weak acid or bicarbonate or a mixture thereof.

9. Haemofiltration fluid according to claim 7 or 8, wherein the buffering compound is lactate.

10. Haemofiltration fluid according to claims 7 or 8, comprising:
| | | |
|---|---|---|
| Na⁺ | 140 | mmol/l |
| Mg²⁺ | 0.8 | mmol/l |
| Cl⁻ | 108 | mmol/l |
| K⁺ | 4 | mmol/l |
| Ca²⁺ | 1.2 | mmol/l |
| Glucose | 6 | mmol/l |
| Lactate | 40 | mmol/l |

11. Haemofiltration fluid according to claims 7 or 8, comprising:
| | | |
|---|---|---|
| Na⁺ | 140 | mmol/l |
| Mg²⁺ | 0.8 | mmol/l |
| Cl⁻ | 118 | mmol/l |
| K⁺ | 4 | mmol/l |
| Ca²⁺ | 1.2 | mmol/l |
| Glucose | 6 | mmol/l |
| Bicarbonate | 30 | mmol/l |

12. Concentrate for the preparation of a haemofiltration fluid suitable for use in continuous veno-venous haemofiltration (CVVH), said concentrate comprising an aqueous solution of physiologically acceptable salts containing at least the Na⁺, Cl⁻, Mg²⁺, K⁺ and Ca²⁺ ions and optionally glucose, all in a physiologically acceptable concentration, according to any one of claims 1 - 9, by dilution with water, wherein, after dilution, at the time of administration, the concentrations of K⁺ ions is higher than 3 mmol/l and lower than 5.5 mmol/l and the concentration of Ca²⁺ ions are in a concentration of 0.8 to 1.3 mmol/l.

## Patentansprüche

1. Hämofiltrationsflüssigkeit geeignet zur Verwendung bei kontinuierlicher veno -venöser Hämofiltration (CVVH), die eine wässrige Lösung aus physiologisch verträglichen Salzen umfasst, die zumindest die Na ⁺, Cl⁻, Mg²⁺, K⁺ und Ca²⁺ Ionen und gegebenenfalls Glukose enthält, alle in einer physiologisch verträglichen Konzentration, wobei die Hämofiltrationsflüssigkeit die Ionen K⁺ in einer Konzentration von mehr als 3 mmol/l und weniger als 5,5 mmol/l und Ca²⁺ in einer Konzentration v on 0,8 bis 1,3 mmol/l enthält.

2. Hämofiltrationsflüssigkeit nach Anspruch 1, wobei die K⁺ Konzentration zwischen 3,5 und 5,5 mmol/l, vorzugsweise zwischen 3,5 und 5 mmol/l liegt.

3. Hämofiltrationsflüssigkeit nach einem der vorstehenden Ansprüche, wob ei die Glukosekonzentration zwischen 0 und 15 mmol/l, vorzugsweise zwischen 2 und 10 mmol/l und am stärksten bevorzugt zwischen 3,5 und 8 mmol/l liegt.

4. Hämofiltrationsflüssigkeit nach einem der vorstehenden Ansprüche, wobei die Flüssigkeit die folgend en Bestandteile in dem aufgeführten physiologisch verträglichen Bereich umfasst:
| | |
|---|---|
| Na⁺ | 135-145 mmol/l |
| Mg²⁺ | 0,6-1,0 mmol/l |
| Cl⁻ | 95-120 mmol/l |
| Glukose | 3,5 - 8 mmol/l |
| K⁺ | 3,5 - 5 mmol/l |
| Ca²⁺ | 0,8-1,3 mmol/l |

5. Hämofiltrationsflüssigkeit nach einem der vorstehenden Ansprüche, wobei die Hämofiltrationsflüssigkeit ferner Citrat umfasst.

6. Hämofiltrationsflüssigkeit nach Anspruch 5, wobei die Citratkonzentration zwischen 10 und 15 mmol/l liegt.

7. Hämofiltrationsflüssigkeit nach einem der Ansprüche 1 bis 4, wobei die Flüssigkeit ferner zumindest eine Pufferverbindung umfasst.

8. Hämofiltrationsflüssigkeit nach Anspruch 7, wobei die zumindest eine Pufferverbindung eine schwache Säure oder Bikarbonat oder eine Mischung davon ist.

9. Hämofiltratio nsflüssigkeit nach Anspruch 7 oder 8, wobei die Pufferverbindung Laktat ist.

10. Hämofiltrationsflüssigkeit nach Ansprüchen 7 oder 8, umfassend:
| | |
|---|---|
| Na⁺ | 140 mmol/l |
| Mg²⁺ | 0,8 mmol/l |
| Cl⁻ | 108 mmol/l |
| K⁺ | 4 mmol/l |
| Ca²⁺ | 1,2 mmol/l |
| Glukose | 6 mmol/l |
| Laktat | 40 mmol/l |

11. Hämofiltrationsflüssigkeit nach Ansprüchen 7 oder 8, umfassend:
| | |
|---|---|
| Na⁺ | 140 mmol/l |
| Mg²⁺ | 0,8 mmol/l |
| Cl⁻ | 118 mmol/l |
| K⁺ | 4 mmol/l |
| Ca²⁺ | 1,2 mmol/l |
| Glukose | 6 mmol/l |
| Bicarbonat | 30 mmol/l |

12. Konzentrat zur Herstellung einer Hämofiltrationsflüss igkeit, die geeignet zur Verwendung bei kontinuierlicher veno -venöser Hämofiltration (CVVH) ist, wobei das Konzentrat eine wässrige Lösung aus physiologisch verträglichen Salzen umfasst, die zumindest die Na⁺, Cl⁻, Mg²⁺, K⁺ und Ca²⁺ Ionen und gegebenenfalls Glukose enthält, alle in einer physiologisch verträglichen Konzentration, nach einem der Ansprüche 1 bis 9, durch Verdünnung mit Wasser, wobei nach der Verdünnung zum Zeitpunkt der Verabreichung die Konzentrationen der K⁺ Ionen höher ist als 3 mmol/l und geringer ist als 5,5 mmol/l und die Konzentration der Ca²⁺ Ionen in einer Konzentration von 0,8 bis 1,3 mmol/l liegt.

## Revendications

1. Fluide d'hémofiltration apte à être utilisé en hémofiltration continue veine à veine (CVVH) comprenant une solution aqueuse de sels physiologiquement acceptables contenant au moins les ions Na⁺, Cl⁻, Mg²⁺, K⁺ et Ca²⁺ et éventuellement du glucose, tous en concentration physiologiquement acceptable, dans lequel le fluide d'hémofiltration contient des ions K⁺ en concentration supérieure à 3 mmol/l et inférieure à 5,5 mmol/l et des ions Ca²⁺ en concentration comprise entre 0,8 et 1,3 mmol/l.

2. Fluide d'hémofiltration selon la revendication 1, dans lequel la concentration en K⁺ se situe entre 3,5 et 5,5 mmol/l, de préférence entre 3,5 et 5 mmol/l.

3. Fluide d'hémofiltration selon l'une quelconque des revendications précédentes, dans lequel la concentration en glucose se situe entre 0 et 15 mmol/l, de préférence entre 2 et 10 mmol/l et plus préférentiellement encore entre 3,5 et 8 mmol/l.

4. Fluide d'hémofiltration selon l'une quelconque des revendications précédentes, dans lequel le fluide contient les composés suivants dans la gamme physiologiquement acceptable indiquée:
| | | |
|---|---|---|
| Na⁺ | 135 - 145 | mmol/l |
| Mg²⁺ | 0,6- 1, 0 | mmol/l |
| Cl⁻ | 95- 120 | mmol/l |
| Glucose | 3,5- 8 | mmol/l |
| K⁺ | 3,5- 5 | mmol/l |
| Ca²⁺ | 0,8 - 1, 3 | mmol/l |

5. Fluide d'hémofiltration selon l'une quelconque des revendications précédentes, dans lequel le fluide d'hémofiltration contient en outre du citrate.

6. Fluide d'hémofiltration selon la revendication 5, dans lequel la concentration en citrate se situe entre 10 et 15 mmol/l.

7. Fluide d'hémofiltration selon l'une quelconque des revendications 1 à 4, dans lequel le fluide contient en outre au moins un composé tampon.

8. Fluide d'hémofiltration selon la revendication 7, dans lequel l'au moins un composé tampon est un acide faible ou du bicarbonate ou un mélange des deux.

9. Fluide d'hémofiltration selon la revendication 7 ou 8, dans lequel le composé tampon est du lactate.

10. Fluide d'hémofiltration selon les revendications 7 ou 8, contenant :
| | | |
|---|---|---|
| Na⁺ | 140 | mmol/l |
| Mg²⁺ | 0, 8 | mmol/l |
| Cl⁻ | 108 | mmol/l |
| K⁺ | 4 | mmol/l |
| Ca²⁺ | 1,2 | mmol/l |
| Glucose | 6 | mmol/l |
| Lactate | 40 | mmol/l |

11. Fluide d'hémofiltration selon les revendications 7 ou 8, cotenant :
| | | |
|---|---|---|
| Na⁺ | 140 | mmol/l |
| Mg²⁺ | 0, 8 | mmol/l |
| Cl⁻ | 118 | mmol/l |
| K⁺ | 4 | mmol/l |
| Ca²⁺ | 1,2 | mmol/l |
| Glucose | 6 | mmol/l |
| Bicarbonate | 30 | mmol/l |

12. Concentré pour la préparation, par dilution avec de l'eau, d'un fluide d'hémofiltration apte à être utilisé en hémofiltration continue veine à veine (CVVH), concentré comprenant une solution aqueuse de sels physiologiquement acceptables contenant au moins les ions Na⁺, Cl⁻, Mg²⁺, K⁺ et Ca²⁺ et éventuellement du glucose, tous en concentration physiologiquement acceptable, selon l'une quelconque des revendications 1 à 9, dans lequel, après dilution, au moment de l'administration, la concentration en ions K⁺ est supérieure à 3 mmol/l et inférieure à 5,5 mmol/l et la concentration en ions Ca²⁺ correspond à une concentration comprise entre 0,8 et 1,3 mmol/l.
